(19) Europäisches Patentamt
European Patent Office
Office européen des brevets

(11) Veröffentlichungsnummer: **0 130 524**
**B1**

(12) # EUROPÄISCHE PATENTSCHRIFT

(45) Veröffentlichungstag der Patentschrift: **23.08.89**

(51) Int. Cl.⁴: **A 61 K 31/195**, A 61 K 31/245, A 61 K 31/455, A 61 K 47/00

(21) Anmeldenummer: **84107267.1**

(22) Anmeldetag: **25.06.84**

(54) **Depot-Antiphlogistika.**

(30) Priorität: **05.07.83 DE 3324192**

(43) Veröffentlichungstag der Anmeldung: **09.01.85 Patentblatt 85/2**

(45) Bekanntmachung des Hinweises auf die Patenterteilung: **23.08.89 Patentblatt 89/34**

(84) Benannte Vertragsstaaten: **AT BE CH DE FR GB IT LI LU NL SE**

(56) Entgegenhaltungen:
**EP-A-0 034 270**
**DE-A-3 026 402**

**CHEMICAL ABSTRACTS, Band 99, Nr.4, 25. Juli 1983, Seite 342, Ref. Nr. 28030c; Columbus, Ohio, US**
**REMINGTON'S PHARMACEUTICAL SCIENCES; 14. Auflage, 1970, "Parenteral preparations", Seite 1523; Mack Publishing Company, Easton, US**

(73) Patentinhaber: **Troponwerke GmbH & Co. KG, Berliner Strasse 156, D-5000 Köln 80 (DE)**

(72) Erfinder: **Dell, Hans- Dieter, Dr., Kalmüntener Strasse 5, D-5060 Berg.- Gladbach 2 (DE)**
Erfinder: **Pelster, Bernhard, Dr., Pleisufer 6a, D-5205 St. Augustin 1 (DE)**
Erfinder: **Kraus, Reinhold, Paffendorf- Strasse 77, D-5000 Köln 91 (DE)**
Erfinder: **Schierstedt, Detlef, Dr., Henri- Dumont-Strasse 2, D-5205 St. Augustin 3 (DE)**

(74) Vertreter: **Adrian, Albert, Dr., c/o BAYER AG Konzernverwaltung RP Patentabteilung, D-5090 Leverkusen 1, Bayerwerk (DE)**

**EP 0 130 524 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die vorliegende Erfindung betrifft Depotzubereitungen zur intramuskulären Anwendung, enthaltend mindestens einen antiphlogistisch/analgetischen Wirkstoff und ein Medium.

Antiphlogistisch-analgetisch Wirkstoffe werden bisher in oraler, rektaler, cutaner sowie intramuskulärer Applikationsweise bei Mensch und Tier angewendet. Hierbei ist die biologische Halbwertzeit und die Wirkdauer für das Applikationsschema ausschlaggebend. Bei zahlreichen dieser Medikamente muß eine tägliche Mehrfachapplikation erfolgen.

Zur Verlängerung der Wirkdauer sind zahlreiche Wirkstoffe in spezieller galenischer Verarbeitung als Retard-Präparate beschrieben, z. B. 1-(p-Chlorbenzoyl)-5-methoxy-2-methylindol-3-essigsäure als sustained release-Präparat in US-PS-4 173 626.

Es gibt bisher kein Antiphlogistikum/Analgetikum in Depot-Form für eine intramuskuläre Anwendung, welches bei Applikation in mehrtägigen Abständen noch eine ausreichende Wirkung entfaltet.

Aufgabe der vorliegenden Erfindung war es daher, ein Antiphlogistikum/Analgetikum in Depot-Form für eine intramuskuläre Anwendung bereitzustellen.

Diese Aufgabe wird gelöst durch Depotzubereitungen für eine intramuskuläre Anwendung enthaltend mindestens einen Wirkstoff der Formel (I)

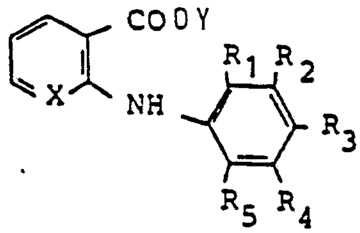

(I)

in der

- $R^1$ - $R^5$ für Wasserstoff, Alkyl mit 1 bis 4 C-Atomen, Trihalogenalkyl,
- X für Stickstoff oder eine CH-Gruppe und
- Y für Wasserstoff, Metallionen, Alkyl mit 1 bis 4 C-Atomen, Alkoxy, Alkoxyalkyl, Hydroxyalkyl, Hydroxyalkoxyalkyl, Trihalogenalkyl, wobei jeweils die Anzahl der C-Atome zwischen 1 und 6 liegt und die Alkylkette 1 bis 4 C-Atome aufweist und gerade oder verzweigt sein kann,

steht,

und als Medium eine oder mehrere Verbindungen aus der Gruppe der Mono-, Di- und Triglyceride mit Mono-, Di- und Tricarbonsäuren der Kettenlänge $C_2$ bis $C_{30}$, in gesättigter wie ungesättigter, gegebenenfalls auch hydroxylierter Form und/oder Ester ein oder mehrwertiger Alkohole der Kettenlänge $C_2$ bis $C_{30}$ mit den genannten Säurekomponenten.

Die Depotzubereitungen enthalten mindestens eine der Verbindungen der allgemeinen Formel I sowie ein Medium. Unter Medium im Sinne der vorliegenden Erfindung werden verstanden:

Mono-, Di- und Triglyceride mit Mono-, Di- und Tricarbonsäuren der Kettenlänge $C_2$-$C_{30}$, in gesättigter wie ungesättigter, gegebenenfalls auch hydroxylierter Form (insbesondere ölige Triglyceride wie Viscoleo, Baumwollsaat-, Erdnuß-, Maiskeim-, Mandel-, Oliven-, Ricinus-, Sesamöl). Ferner sind geeignet Ester ein- oder mehrwertiger Alkohole, z. B. Propylenglykol, Butandiole und höhere Alkanole, Alkandiole, der Kettenlänge $C_2$-$C_{30}$ mit den oben erwähnten Säurekomponenten (als Beispiele seien genannt: Ethyloleat, Isopropylmyristat, Isopropylstearat), sowie gegebenenfalls die entsprechenden zuvor erwähnten Alkohole selbst.

Das Medium kann anstelle oder neben geeigneten Estern auch Ether, Alkohole und Amide (gesättigt wie ungesättigt, aliphatisch, aromatisch) enthalten, die Amide können auch cyclisiert sein, z. B. Pyrrolidon-(2) (als Monomers oder auch Polymeres). Diese können auch polyfunktionell sein, z. B. Polyole, Mono- und Diketale, Acetale, Polyether, cyclische Ether etc., welche toxikologisch indifferent sind.

Ganz besonders bevorzugt enthalten die Depotzubereitungen als Medium mindestens eins aus der Gruppe:

Viscoleo,
Isopropylmyristat,
Ethyloleat,
Ricinusöl,
Sesamöl,
Arachisöl,
Baumwollsamenöl,
Mandelöl,
Olivenöl,
Klauenöl,
Neutralöl und
Maisöl.

Unter Viscoleo wird ein Gemisch von Triglyceride, gesättigter Fettsäuren mittlerer Kettenlänge verstanden,

das neutral und bei Zimmertemperatur flüssig ist. Insbesondere wird unter Viscoleo ein Gemisch verstanden, das der in Fiedler, "Lexikon der Hilfsstoffe für Pharmazie, Kosmetik und angrenzende Gebiete", 2. Auflage, 1981, Seite 993 gegebenen Definiton entspricht.

Unter Klauenöl wird das aus den Klauen von Rindern, Hammeln oder den Füßen von Pferden durch Auskochen mit Wasser erhaltende, hellgelb gefärbte, nur wenig riechende Öl verstanden, entsprechend oder Definition bei H.P. Fiedler "Lexikon der Hilfsstoffe für Pharmazie, Kosmetik und angrenzende Gebiete", 2. Aufl. Seite 790 (1981). Neutralöl, z. B. Miglyol, ist nach Fiedler (s.o., S. 615), Capryl-Caprinsäure-Triglycerid, eine Flüssigkeit mit niedriger Viskosität.

Der Anteil des Mediums in der erfindungsgemäßen Depotzubereitung liegt zwischen 2 und 90 %, vorzugsweise 3 bis 70 %, besonders bevorzugt 10 bis 60 %.

Die sterilen Lösungen oder Suspensionen für Injektionszwecke können zusätzlicher Geliermittel, z. B. Aluminiumstearate enthalten, und die Liberation aus dem Öldepot zu verzögern.

Gegebenenfalls können ein oder mehrere Konservierungsmittel verwendet werden, z. B. Benzylalkohol, Phenylethylalkohol, Chlorbutanol, Phenolderivate, z. B. Chlorkresol.

Gegebenenfalls werden dem Medium ein oder mehrere Antioxidantien zugesetzt, z. B. Tokopherole, Nordihydroguajaretsäure, Anisol-Derivate, Ascorbin-, Gallussäureester, Butylhydroxytoluole.

Die Depotzubereitung kann gegebenenfalls auch in zunächst getrennter Form (Wirkstoff/Lösungs- bzw. Suspensionsmedium) vorliegen und vor Gebrauch vereinigt werden (z. B. Trockenampulle).

Bevorzugt werden als Wirkstoffe solche Verbindungen der allgemeinen Formel I verstanden, in welcher:

$R_3$ und $R_4$ für Wasserstoff,
$R_1$, $R_2$, $R_5$ für Methyl, Trifluormethyl, Chlor,
X für eine CH-Gruppe und
Y für Wasserstoff, Metallionen, Alkyl mit 1 bis 4 C-Atomen sowie durch substituiertes Alkyl steht.

Besonders bevorzugt werden unter den erfindungsgemäßen Depotzubereitungen solche verstanden, die als Wirkstoff mindestens eine der folgenden Verbindungen enthalten:

1. N-(α,α,α-Trifluor-m-tolyl)-anthranilsäure

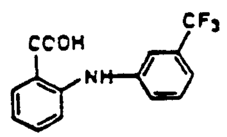

2. N-(2,3-Xylyl)-anthranilsäure

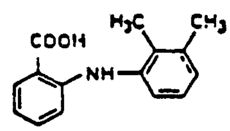

3. N-(2,6-Dichlor-m-tolyl)-anthranilsäure

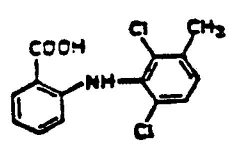

4. N-(3-Chlor-o-tolyl)-anthranilsäure

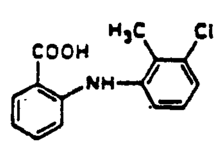

5. N-(2,3-Dichlorphenyl)-anthranilsäure

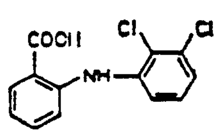

6. N-(α,α,α-Trifluor-m-tolyl)-nicotinsäure

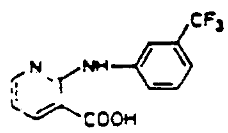

7. N-(2,3-Xylidino)-nicotinsäure

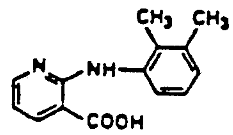

8. 2-(2-Methyl-3-trifluormethylanilino)-nicotinsäure

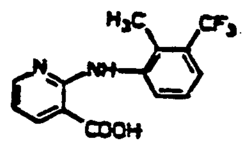

9. N-(3-Chlor-o-tolyl)-nicotinsäure

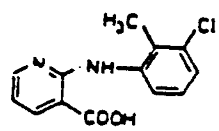

10. 2-(2,6-Xylidino)-nicotinsäure

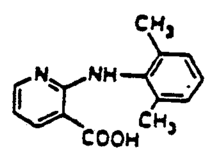

und deren Salze, Ester etc., besonders

11. 2-(2-Hydroxyethoxy)-ethyl-N-(α,α,α-trifluor-m-toluol)-anthranilat

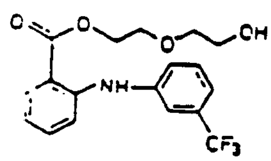

12. Ethoxymethyl-N-(2,6-dichloro-m-tolyl)-anthranilat

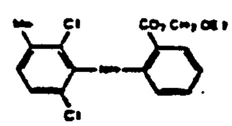

13. Butyl-N-(α,α,α-Trifluor-m-tolyl)-anthranilat

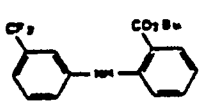

14. Ethyl-N-(α,α,α-Trifluor-m-tolyl)-anthranilat

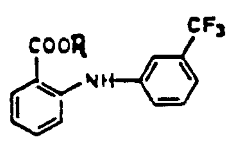

a: R = Ethyl

15. Isopropyl-N-(α,α,α -Trifluor-m-tolyl)-anthranilat

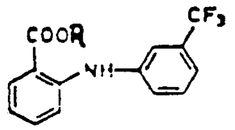 b: R = iso-Propyl

Die pharmakologisch wirksamen Verbindungen der allgemeinen Formel I sind in den erfindungsgemäßen Depotzubereitungen in Mengen von 3 bis 80 Gew.-%, vorzugsweise von 5 bis 75 Gew.-%, besonders bevorzugt von 10 bis 70 Gew.-% enthalten.

Die erfindungsgemäßen Depotzubereitungen können außer den Wirkstoffen der allgemeinen Formel I noch weitere pharmazeutische Wirkstoffe enthalten.

Die Herstellung der vorstehend beschriebenen Zubereitungen erfolgt durch intensives Mischen des oder der Wirkstoffe der allgemeinen Formel I mit dem Suspensionsmedium in den vorstehend angegebenen Mengenverhältnissen.

Die Herstellung von Lösungen erfolgt üblicherweise durch Lösung der keimarmen bzw. sterilen Wirk- und Hilfsstoffe im sterilisierten Lösungsmittel. Anschließend wird steril filtriert und in Ampullen abgefüllt.

Die Suspension wird aus sterilen Wirk- und Hilfsstoffen unter aseptischen Bedingungen bereitet. Gegebenenfalls wird (bei Lösung wie Suspension) unter Schutzgas gearbeitet, Hitzesterilisation ist in besonderen Fällen möglich.

Die Ampullenfüllung beträgt 0,5 - 5,0 ml. Die zu applizierende Lösung bzw. Suspension beträgt bevorzugt 1 - 3 ml, besonders bevorzugt 1 - 2 ml.

Die ödemhemmende und die analgetische Wirkung der Testsubstanzen nach i.m. Applikation einer entsprechenden Depot-Zubereitung wurde mit Hilfe des durch Carrageenan induzierten Ödems der Rattenpfote bzw. mittels des Randall-Selitto-Test [Arch. Int. Pharmacodyn. 111, 409 (1957)] ermittelt.

Die Versuche wurden mit männlichen Ratten [Stamm: Bor: WISW (SPF-Cpb), Gewicht um 200 g] dürchgeführt. Den Tieren wurde einmalig ein Depotpräparat i.m. appliziert und an den folgenden Tagen die entzündungshemmende Wirkung durch Auslösung des Carrageenan-Ödems überprüft. Pro Tag wurden jeweils drei Gruppen von je 5 Tieren untersucht, wobei die erste Gruppe aus 5 unbehandelten, die 2. Gruppe aus 5 nur mit Lösungsmittel (z. B. Viscoleo) behandelten und die 3. Gruppe aus S mit Depot-Präparat (z. B. 2-(2-Hydroxyethoxy)-ethyl-N-(α,α,α-trifluor-m-tolyl)anhtranilat (Formel 11) in Viscoleo) behandelten Tiere bestand.

Die Pfotenvolumen wurden nach der Methode von F. Kemper und G. Ameln [Z. ges. exp. Med. 131, 407 (1959)] gemessen, wobei die Differenz von Pfotenvolumen 5 Stunden nach der Ödemprovokation und dem normalen Pfotenvolumen das Ödemvolumen ergibt. Die analgetische Wirkung wurde über den Druckschmerz ermittelt [Randall-Selitto, s.o.].

Die Hemmung des Carrageenan-Ödems nach i.m. Applikation von 2-(2-Hydroxyethoxy)-ethyl-N-(α,α,α-trifluor-m-tolyl)-anthranilat (Formel 11) in Viscoleo beträgt nach verschiedenen Dosen bei konstantem Applikationsvolumen von 0,03 ml (Ratte):

| Dosis mg/kg | Tag post injectionem | Ödemhemmung (%) | Steigerung der Schmerzbelastung (%) |
|---|---|---|---|
| 10,5 | 1 | 64,0 | |
| | 2 | 61,1 | |
| | 3 | 36,6 | |
| | 4 | 41,6 | |
| 15 | 1 | 77,6 | 64,3 (1 h) |
| | 2 | 72,2 | 75,3 (24 h) |
| | 3 | 55,5 | 46,6 (48 h) |
| | 4 | 51,3 | 32,9 (72 h) |
| 19,5 | 1 | 77,6 | |
| | 2 | 81,3 | |
| | 3 | 61,6 | |
| | 4 | 52,1 | |

P.o.-Applikation von 2-(2-Hydroxyethoxy)-ethyl-N-(α,α,α-trifluor-m-tolyl)-anthranilat führt beim gleichen Modell (Carrageenan) zu starker Ödemhemmung, welche jedoch nach ca. 24 Stunden dem Kontrollwert

entspricht [H. Jacobi et al., Arzneimittelforsch. 27, 1328 (1977)].

Die Ödemhemmung nach i.m. Applikation von Butyl-N-(α,α,α-trifluor-m-tolyl)-anthranilat (Formel 13) in Viscoleo betrug 1, 2, 3, 4 bzw. 5 Tage nach einmaliger i.m. Gabe von 15 mg/kg 53,8, 46,5, 34,3, 37,1 bzw. 28,8 %.

Die folgenden Säuren erzielten nach i.m. Applikation von 15 mg/kg als ölige Lösung folgende Hemmung:

| Tag post injectionem | % Hemmung nach Depot-Säure N-(α,α,α-Trifluor-m-tolyl)-anthranilsäure (Formel 1) | N-(α,α,α-Trifluor-m-tolyl)-nicotinsäure (Formel 6) |
|---|---|---|
| 1 | 53,8 | 57,0 |
| 2 | 46,5 | 35,2 |
| 3 | 34,3 | 35,5 |
| 4 | 37,1 | 11,5 |
| 5 | 26,8 | |

Bei Ratten konnte die biologische Halbwertszeit der Verbindung 11 aus dem entzündeten Gewebe heraus mittels Gaschromatographie bestimmt werden [H.-D. Dell, J. Fiedler, H. Jacobi und J. Kolle, Arzheim.-Forsch./Drug.Res 31, 17 - 21 (1981)]. Die HWZ aus dem Gewebe beträgt ca. 8,5 Std. Dagegen wird nach i.m. Applikation von 11 in öliger Lösung eine HWZ von 1.29 Tagen für die Elimination aus dem Gewebe (Applikationsort) ermittelt.

Zur vorliegenden Erfindung gehört auch die Verwendung der Depot-Präparate in der Human- und Veterinärmedizin bei der Bekämpfung von entzündlichen und/oder schmerzhaften Prozessen.

Im allgemeinen empfiehlt es sich sowohl in der Human- als auch in der Veterinärmedizin, die erfindungsgemäßen Wirkstoffe in Gesamtmengen von etwa 0,1 bis etwa 100, vorzugsweise 0,3 bis 10 mg/kg Körpergewicht je Injektion zu verabreichen. Es kann jedoch erforderlich sein, von den genannten Dosierungen abzuweichen und zwar in Abhängigkeit von der Art und dem Körpergewicht des zu behandelden Objektes und der Art und der Schwere der Erkrankung.

Die folgenden Beispiele veranschaulichen erfindungsgemäße, injizierbare, ölige Lösungen bzw. Suspensionen, die als Depot-Antiphlogistika bzw. Depot-Amalgetika eingesetzt werden können und besonders bevorzugte Zusammensetzungen darstellen:

| | | |
|---|---|---|
| A 1 | 2-(2-Hydroxyethoxy)-ethyl-N-(α,α,α-trifluor-m-tolyl)-anthranilat (Formel 11) | 500 g |
| | Viscoleo | ad 1000 g |
| 2 | 2-(2-Hydroxyethoxy)-ethyl-N-(α,α,α-trifluor-m-tolyl)-anthranilat (Formel 11) | 500 g |
| | Isopropylmyristat | ad 1000 g |
| 3 | 2-(2-Hydroxyethoxy)-ethyl-N-(α,α,α-trifluor-m-tolyl)-anthranilat (Formel 11) | 400 g |
| | Gallussäurepropylester | 10 g |
| | Ethyloleat | ad 1000 g |
| 4 | 2-(2-Hydroxyethoxy)-ethyl-N-(α,α,α-trifluor-m-tolyl)-anthranilat (Formel 11) | 400 g |
| | Miglyol 812 | ad 1000 g |
| B 1 | Butyl-N-(α,α,-trifluor-m-tolyl)-anthranilat (Formel 13) | 500 g |
| | Viscoleo | ad 1000 g |
| B 2 | Ethyl-(α,α,α-trifluor-m-tolyl)-anthranilat (Formel 14) | 500 g |
| | Isopropylmyristat | ad 1000 g |
| 3 | Isopropyl-(α,α,α-trifluor-m-tolyl)-anthranilat (Formel 15) | 490 g |
| | Gallussäurepropylester | 10 g |
| | Ethyloleat | ad 1000 g |
| 4 | Butyl-(α,α,α-trifluor-m-tolyl)-anthranilat (Formel 13) | 600 g |
| | Miglyol 812 | ad 1000 g |
| C 1 | N-(α,α,α-Trifluor-m-tolyl)-anthranilsäure (Formel 1) | 170 g |
| | Aluminiumstearat | 20 g |
| | Miglyol 812 | ad 1000 |
| 2 | N-(2,3-Xylyl)-anthranilsäure (Formel 2) | 170 g |
| | Aluminiumstearat | 15 g |
| | Viscoleo | ad 1000 g |

| | | |
|---|---|---|
| 3 | N-(α,α,α-Trifluor-m-tolyl)-nicotinsäure (Formel 6) | 300 g |
| | Gallussäurepropylester | 10 g |
| | Ricinusöl | ad 1000 g |
| 4 | N-(2,6-Dichlor-m-tolyl)-anthranilsäure | 400 g |
| | Gallussäurepropylester (Formel 3) | 10 g |
| | Sesamöl | ad 1000 g |

| | |
|---|---|
| Arachisöl | = Ol. arachidis neutralisatum |
| Baumwollsamenöl | = Ol. gossypii neutralisatum |
| Mandelöl | = Ol. amygdalae neutralisatum |
| Olivenöl | = Ol. olivae neutralisatum |
| Sesamöl | = Ol. sesami neutralisatum |
| Ricinusöl | = Ol. ricini neutralisatum |
| Klauenöl | = Ol. pedis bovis/equi |
| Neutralöl | = Ol. neutrale (z. B. Miglyol) |
| Maisöl | = Ol. maydis neutralisatum |

Miglyol 812 ist ein Capryl-Caprinsäure-Triglycerid, vgl. Fiedler, Lexikon der Hilfsstoffe, Editro Canter, Aulendorf, 2. Aufl., 1981, Seite 616.

**Tabelle 1**

| Beispiel | Wirkstoff: N-(α,α,α-Trifluor-m-tolyl)-anthranilsäure (1) [mg] | Lösungs- bzw. Suspensionsmedium [ad 1000 mg] | Gallussäure-propylester [mg] | Al-Stearat [mg] | Neutralöl [mg] |
|---|---|---|---|---|---|
| 1 | 300 | Arachisöl | 10 | | |
| 2 | 120 | " | 10 | | |
| 3 | 250 | Baumwollsamenöl | | | |
| 4 | 100 | " | | 20 | |
| 5 | 250 | Mandelöl | | | |
| 6 | 100 | " | | | |
| 7 | 300 | Maisöl | | | |
| 8 | 120 | " | | | |
| 9 | 300 | Olivenöl | 10 | | |
| 10 | 120 | " | 10 | | |
| 11 | 250 | Rizinusöl | 10 | | |
| 12 | 100 | " | 10 | | |
| 13 | 250 | Sesamöl | | | |
| 14 | 100 | " | | | |
| 15 | 300 | Klauenöl | | 20 | |
| 16 | 120 | " | | | |
| 17 | 300 | Neutralöl | | 20 | |

**Tabelle 2**

| Beispiel | Wirkstoff: N-(2,3-Xylyl)-anthranilsäure (2) [mg] | Lösungs- bzw. Suspensionsmedium [ad 1000 mg] | Gallussäure-propylester [mg] | Al-Stearat [mg] | Neutralöl [mg] |
|---|---|---|---|---|---|
| 1 | 300 | Arachisöl | 10 | | |
| 2 | 120 | " | 10 | | |
| 3 | 250 | Baumwollsamenöl | | | |
| 4 | 100 | " | | 20 | |
| 5 | 250 | Mandelöl | | | |
| 6 | 100 | " | | | |
| 7 | 300 | Maisöl | | | |
| 8 | 120 | " | | | |
| 9 | 300 | Olivenöl | 10 | | |
| 10 | 120 | " | 10 | | |
| 11 | 250 | Rizinusöl | 10 | | |
| 12 | 100 | " | 10 | | |
| 13 | 250 | Sesamöl | | | |
| 14 | 100 | " | | | |

| | | | | | |
|---|---|---|---|---|---|
| 15 | 300 | Klauenöl | | 20 | |
| 16 | 120 | " | | | |
| 17 | 300 | Neutralöl | | 20 | |

**Tabelle 3**

| Beispiel | Wirkstoff: N-(2,6-Dichlor-m-tolyl)-anthranilsäure (3) [mg] | Lösungs- bzw. Suspensionsmedium [ad 1000 mg] | Gallussäurepropylester [mg] | Al-Stearat [mg] | Neutralöl [mg] |
|---|---|---|---|---|---|
| 1 | 300 | Ethyloleat | | | |
| 2 | 120 | " | | | |
| 3 | 250 | Isoprypylmyristat | | | |
| 4 | 100 | " | | 20 | |
| 5 | 250 | Isopropylpalmitat | | | |
| 6 | 120 | " | | | |
| 7 | 300 | Oleyloleat | | | |
| 8 | 100 | " | | | |
| 9 | 250 | Benzylbenzoat | | | 300 |
| 10 | 100 | " | | | 400 |
| 11 | 300 | Mohnöl | 10 | | |
| 12 | 120 | " | 10 | | |
| 13 | 300 | Viscoleo | | | |
| 14 | 120 | " | | 20 | |

**Tabelle 4**

| Beispiel | Wirkstoff: N-(3,Chlor-o-tolyl)-anthranilsäure (4) [mg] | Lösungs- bzw. Suspensionsmedium [ad 1000 mg] | Gallussäurepropylester [mg] | Al-Stearat [mg] | Neutralöl [mg] |
|---|---|---|---|---|---|
| 1 | 300 | Arachisöl | 10 | | |
| 2 | 120 | " | 10 | | |
| 3 | 250 | Baumwollsamenöl | | | |
| 4 | 100 | " | | 20 | |
| 5 | 250 | Mandelöl | | | |
| 6 | 100 | " | | | |
| 7 | 300 | Maisöl | | | |
| 8 | 120 | " | | | |
| 9 | 300 | Olivenöl | 10 | | |
| 10 | 120 | " | 10 | | |
| 11 | 250 | Rizinusöl | 10 | | |
| 12 | 100 | " | 10 | | |
| 13 | 250 | Sesamöl | | | |
| 14 | 100 | " | | | |
| 15 | 300 | Klauenöl | | 20 | |
| 16 | 120 | " | | | |
| 17 | 300 | Neutralöl | | 20 | |

**Tabelle 5**

| Beispiel | Wirkstoff: N-(2,3-Dichlorphenyl)-anthranilsäure (5) [mg] | Lösungs- bzw. Suspensionsmedium [ad 1000 mg] | Gallussäurepropylester [mg] | Al-Stearat [mg] | Neutralöl [mg] |
|---|---|---|---|---|---|
| 1 | 300 | Ethyloleat | | | |
| 2 | 120 | " | | | |
| 3 | 250 | Isoprypylmyristat | | | |
| 4 | 100 | " | | 20 | |
| 5 | 250 | Isopropylpalmitat | | | |
| 6 | 120 | " | | | |
| 7 | 300 | Oleyloleat | | | |
| 8 | 100 | " | | | |
| 9 | 250 | Benzylbenzoat | | | 300 |

| | | | | | |
|---|---|---|---|---|---|
| 10 | 100 | " | | | 400 |
| 11 | 300 | Mohnöl | 10 | | |
| 12 | 120 | " | 10 | | |
| 13 | 300 | Viscoleo | | | |
| 14 | 120 | " | | 20 | |

**Tabelle 6**

| Beispiel | Wirkstoff: N-(α,α,α-Trifluor-m-tolyl)-nicotinsäure (6) [mg] | Lösungs- bzw. Suspensionsmedium [ad 1000 mg] | Gallussäure-propylester [mg] | Al-Stearat [mg] | Neutralöl [mg] |
|---|---|---|---|---|---|
| 1 | 300 | Arachisöl | 10 | | |
| 2 | 120 | " | 10 | | |
| 3 | 250 | Baumwollsamenöl | | | |
| 4 | 100 | " | | 20 | |
| 5 | 250 | Mandelöl | | | |
| 6 | 100 | " | | | |
| 7 | 300 | Maisöl | | | |
| 8 | 120 | " | | | |
| 9 | 300 | Olivenöl | 10 | | |
| 10 | 120 | " | 10 | | |
| 11 | 250 | Rizinusöl | 10 | | |
| 12 | 100 | " | 10 | | |
| 13 | 250 | Sesamöl | | | |
| 14 | 100 | " | | | |
| 15 | 300 | Klauenöl | | 20 | |
| 16 | 120 | " | | | |
| 17 | 300 | Neutralöl | | 20 | |

**Tabelle 7**

| Beispiel | Wirkstoff: N-(2,3-Xylidino)-nicotinsäure (7) [mg] | Lösungs- bzw. Suspensionsmedium [ad 1000 mg] | Gallussäure-propylester [mg] | Al-Stearat [mg] | Neutralöl [mg] |
|---|---|---|---|---|---|
| 1 | 300 | Ethyloleat | | | |
| 2 | 120 | " | | | |
| 3 | 250 | Isoprypylmyristat | | | |
| 4 | 100 | " | | 20 | |
| 5 | 250 | Isopropylpalmitat | | | |
| 6 | 120 | " | | | |
| 7 | 300 | Oleyloleat | | | |
| 8 | 100 | " | | | |
| 9 | 250 | Benzylbenzoat | | | 300 |
| 10 | 100 | " | | | 400 |
| 11 | 300 | Mohnöl | 10 | | |
| 12 | 120 | " | 10 | | |
| 13 | 300 | Viscoleo | | | |
| 14 | 120 | " | | 20 | |

**Tabelle 8**

| Beispiel | Wirkstoff: 2-(2-Methyl-3-trifluormethyl-anilino)-nicotinsäure (8) [mg] | Lösungs- bzw. Suspensionsmedium [ad 1000 mg] | Gallussäure-propylester [mg] | Al-Stearat [mg] | Neutralöl [mg] |
|---|---|---|---|---|---|
| 1 | 300 | Arachisöl | 10 | | |
| 2 | 120 | " | 10 | | |
| 3 | 250 | Baumwollsamenöl | | | |
| 4 | 100 | " | | 20 | |
| 5 | 250 | Mandelöl | | | |
| 6 | 100 | " | | | |
| 7 | 300 | Maisöl | | | |
| 8 | 120 | " | | | |

| | | | | | |
|---|---|---|---|---|---|
| 9 | 300 | Olivenöl | 10 | | |
| 10 | 120 | " | 10 | | |
| 11 | 250 | Rizinusöl | 10 | | |
| 12 | 100 | " | 10 | | |
| 13 | 250 | Sesamöl | | | |
| 14 | 100 | " | | | |
| 15 | 300 | Klauenöl | | 20 | |
| 16 | 120 | " | | | |
| 17 | 300 | Neutralöl | | 20 | |

**Tabelle 9**

| Beispiel | Wirkstoff: N-(3-Chlor-o-tolyl)-nicotinsäure (9) [mg] | Lösungs- bzw. Suspensionsmedium [ad 1000 mg] | Gallussäure-propylester [mg] | Al-Stearat [mg] | Neutralöl [mg] |
|---|---|---|---|---|---|
| 1 | 300 | Ethyloleat | | | |
| 2 | 120 | " | | | |
| 3 | 250 | Isoprypylmyristat | | | |
| 4 | 100 | " | | 20 | |
| 5 | 250 | Isopropylpalmitat | | | |
| 6 | 120 | " | | | |
| 7 | 300 | Oleyloleat | | | |
| 8 | 100 | " | | | |
| 9 | 250 | Benzylbenzoat | | | 300 |
| 10 | 100 | " | | | 400 |
| 11 | 300 | Mohnöl | 10 | | |
| 12 | 120 | " | 10 | | |
| 13 | 300 | Viscoleo | | | |
| 14 | 120 | " | | 20 | |

**Tabelle 10**

| Beispiel | Wirkstoff: 2-(2,6-Xylidino)-nicotinsäure (10) [mg] | Suspensionsmedium [ad 1000 mg] | Gallussäure-propylester [mg] | Al-Stearat [mg] | Neutralöl [mg] |
|---|---|---|---|---|---|
| 1 | 300 | Arachisöl | 10 | | |
| 2 | 120 | " | 10 | | |
| 3 | 250 | Baumwollsamenöl | | | |
| 4 | 100 | " | | 20 | |
| 5 | 250 | Mandelöl | | | |
| 6 | 100 | " | | | |
| 7 | 300 | Maisöl | | | |
| 8 | 120 | " | | | |
| 9 | 300 | Olivenöl | 10 | | |
| 10 | 120 | " | 10 | | |
| 11 | 250 | Rizinusöl | 10 | | |
| 12 | 100 | " | 10 | | |
| 13 | 250 | Sesamöl | | | |
| 14 | 100 | " | | | |
| 15 | 300 | Klauenöl | | 20 | |
| 16 | 120 | " | | | |
| 17 | 300 | Neutralöl | | 20 | |

**Tabelle 11**

| Beispiel | Wirkstoff: 2-(2-Hydroxyethoxy)-ethyl-N-(α,α,α-trifluor-m-tolyl)-anthranilat (11) [mg] | Lösungs- bzw. Suspensionsmedium [ad 1000 mg] | Gallussäure-propylester [mg] | Al-Stearat [mg] | Neutralöl [mg] |
|---|---|---|---|---|---|
| 1 | 450 | Arachisöl | 10 | | |
| 2 | 250 | " | 10 | | |
| 3 | 500 | Baumwollsamenöl | | 20 | |

| | | | | | |
|---|---|---|---|---|---|
| 4 | 250 | " | | | |
| 5 | 480 | Mandelöl | | | |
| 6 | 200 | " | | | |
| 7 | 500 | Maisöl | | | |
| 8 | 250 | " | | | |
| 9 | 400 | Olivenöl | 10 | | |
| 10 | 250 | " | 10 | | |
| 11 | 400 | Rizinusöl | 10 | | |
| 12 | 200 | " | 10 | | |
| 13 | 450 | Sesamöl | | | |
| 14 | 220 | " | | | |
| 15 | 500 | Klauenöl | | | |
| 16 | 250 | " | | 20 | |
| 17 | 550 | Neutralöl | | | |
| 18 | 300 | " | | 20 | |
| 19 | 500 | Ethyloleat | | | |
| 20 | 250 | " | | | |
| 21 | 550 | Isopropylmyristat | | | |
| 22 | 300 | " | | 20 | |
| 23 | 550 | Isopropylpalmitat | | | |
| 24 | 300 | " | | | |
| 25 | 500 | Oleyloleat | | | |
| 26 | 250 | " | | | |
| 27 | 450 | Benzylbenzoat | | | 250 |
| 28 | 200 | " | | | 500 |
| 29 | 450 | Mohnöl | 10 | | |
| 30 | 250 | " | 10 | | |
| 31 | 500 | Viscoleo | | | |
| 32 | 250 | " | | 20 | |

**Tabelle 12**

| Beispiel | Wirkstoff: Ethoxymethyl-N-(2,6-dichloro-m-tolyl)-anthranilat (12) [mg] | Lösungs- bzw. Suspensionsmedium [ad 1000 mg] | Gallussäure-propylester [mg] | Al-Stearat [mg] | Neutralöl [mg] |
|---|---|---|---|---|---|
| 1 | 450 | Arachisöl | 10 | | |
| 2 | 250 | " | 10 | | |
| 3 | 500 | Baumwollsamenöl | | 20 | |
| 4 | 250 | " | | | |
| 5 | 480 | Mandelöl | | | |
| 6 | 200 | " | | | |
| 7 | 500 | Maisöl | | | |
| 8 | 250 | " | | | |
| 9 | 400 | Olivenöl | 10 | | |
| 10 | 250 | " | 10 | | |
| 11 | 400 | Rizinusöl | 10 | | |
| 12 | 200 | " | 10 | | |
| 13 | 450 | Sesamöl | | | |
| 14 | 220 | " | | | |
| 15 | 500 | Klauenöl | | | |
| 16 | 250 | " | | 20 | |
| 17 | 550 | Neutralöl | | | |
| 18 | 300 | " | | 20 | |
| 19 | 500 | Ethyloleat | | | |
| 20 | 250 | " | | | |
| 21 | 550 | Isopropylmyristat | | | |
| 22 | 300 | " | | 20 | |
| 23 | 550 | Isopropylpalmitat | | | |
| 24 | 300 | " | | | |
| 25 | 500 | Oleyloleat | | | |
| 26 | 250 | " | | | |
| 27 | 450 | Benzylbenzoat | | | 250 |
| 28 | 200 | " | | | 500 |
| 29 | 450 | Mohnöl | 10 | | |

| | | | | | |
|---|---|---|---|---|---|
| 30 | 250 | ″ | 10 | | |
| 31 | 500 | Viscoleo | | | |
| 32 | 250 | ″ | | 20 | |

**Tabelle 13**

| Beispiel | Wirkstoff: Butyl-(α,α,α-trifluor-m-tolyl)-anthranilat (13) [mg] | Lösungs- bzw. Suspensionsmedium [ad 1000 mg] | Gallussäurepropylester [mg] | Al-Stearat [mg] | Neutralöl [mg] |
|---|---|---|---|---|---|
| 1 | 450 | Arachisöl | 10 | | |
| 2 | 250 | ″ | 10 | | |
| 3 | 500 | Baumwollsamenöl | | 20 | |
| 4 | 250 | ″ | | | |
| 5 | 480 | Mandelöl | | | |
| 6 | 200 | ″ | | | |
| 7 | 500 | Maisöl | | | |
| 8 | 250 | ″ | | | |
| 9 | 400 | Olivenöl | 10 | | |
| 10 | 250 | ″ | 10 | | |
| 11 | 400 | Rizinusöl | 10 | | |
| 12 | 200 | ″ | 10 | | |
| 13 | 450 | Sesamöl | | | |
| 14 | 220 | ″ | | | |

**Tabelle 14**

| Beispiel | Wirkstoff: Ethyl-(α,α,α-trifluor-m-tolyl)-anthranilat (14a) [mg] | Lösungs- bzw. Suspensionsmedium [ad 1000 mg] | Gallussäurepropylester [mg] | Al-Stearat [mg] | Neutralöl [mg] |
|---|---|---|---|---|---|
| 1 | 500 | Klauenöl | | | |
| 2 | 250 | ″ | | 20 | |
| 3 | 550 | Neutralöl | | | |
| 4 | 300 | ″ | | 20 | |
| 5 | 500 | Ethyloleat | | | |
| 6 | 250 | ″ | | | |
| 7 | 550 | Isopropylmyristat | | | |
| 8 | 300 | ″ | | 20 | |
| 9 | 550 | Isopropylpalmitat | | | |
| 10 | 300 | ″ | | | |
| 11 | 500 | Oleyloleat | | | |
| 12 | 250 | ″ | | | |
| 13 | 450 | Benzylbenzoat | | | |
| 14 | 200 | ″ | | | |
| 15 | 450 | Mohnöl | 10 | | |
| 16 | 250 | ″ | 10 | | |
| 17 | 500 | Viscoleo | | | |
| 18 | 250 | ″ | | 20 | |

**Tabelle 15**

| Beispiel | Wirkstoff: Isopropyl-(α,α,α-trifluor-m-tolyl)-anthranilat (14b) [mg] | Lösungs- bzw. Suspensionsmedium [ad 1000 mg] | Gallussäurepropylester [mg] | Al-Stearat [mg] | Neutralöl [mg] |
|---|---|---|---|---|---|
| 1 | 500 | Klauenöl | | | |
| 2 | 250 | ″ | | 20 | |
| 3 | 550 | Neutralöl | | | |
| 4 | 300 | ″ | | 20 | |
| 5 | 500 | Ethyloleat | | | |
| 6 | 250 | ″ | | | |
| 7 | 550 | Isopropylmyristat | | | |
| 8 | 300 | ″ | | 20 | |
| 9 | 550 | Isopropylpalmitat | | | |

| | | | | |
|---|---|---|---|---|
| 10 | 300 | " | | |
| 11 | 500 | Oleyloleat | | |
| 12 | 250 | " | | |
| 13 | 450 | Benzylbenzoat | | |
| 14 | 200 | " | | |
| 15 | 450 | Mohnöl | 10 | |
| 16 | 250 | " | 10 | |
| 17 | 500 | Viscoleo | | |
| 18 | 250 | " | | 20 |

**Patentansprüche**

1. Depotzubereitungen für eine intramuskuläre Anwendung enthaltend mindestens einen Wirkstoff der Formel

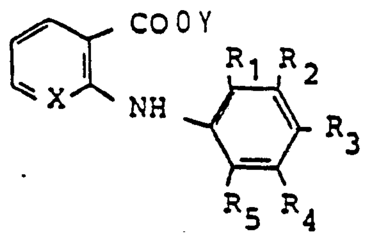

in der
$R^1$ - $R^5$ für Wasserstoff, Alkyl mit 1 bis 4 C-Atomen, Trihalogenalkyl,
X für Stickstoff oder eine CH-Gruppe und
Y für Wasserstoff, Metallionen, Alkyl mit 1 bis 4 C-Atomen, Alkoxy, Alkoxyalkyl, Hydroxyalkyl, Hydroxyalkoxyalkyl, Trihalogenalkyl, wobei jeweils die Anzahl der C-Atome zwischen 1 und 6 liegt und die Alkylkette 1 bis 4 C-Atome aufweist und gerade oder verzweigt sein kann,
steht,
und als Medium eine oder mehrere Verbindungen aus der Gruppe der Mono-, Di- und Triglyceride mit Mono-, Di- und Tricarbonsäuren der Kettenlänge $C_2$ bis $C_{30}$, in gesättigter wie ungesättigter, gegebenenfalls auch hydroxylierter Form und/oder Ester ein oder mehrwertiger Alkohole der Kettenlänge $C_2$ bis $C_{30}$ mit den genannten Säurekomponenten.

2. Depotzubereitungen nach Anspruch 1, enthaltend mindestens einen Wirkstoff der Formel gemäß Anspruch 1,
worin
$R^3$ und $R^4$ für Wasserstoff,
$R^1$, $R^2$, $R^5$ für Methyl, Trifluormethyl, Chlor,
X für eine CH-Gruppe und
Y für Wasserstoff, Metallionen, Alkyl mit 1 bis 4 C-Atomen sowie durch substituiertes Alkyl steht.

3. Depotzubereitung nach Anspruch 1, enthaltend als Wirkstoff eine Verbindung aus der Gruppe
N-(α,α,αTrifluor-m-tolyl)-anthranilsäure
N-(2,3-Xylyl)-anthranilsäure
N-(2,6-Dichlor-m-tolyl)-anthranilsäure
N-(3-Chlor-o-tolyl)-anthranilsäure
N-(2,3-Dichlorphenyl)-anthranilsäure
N-(α,α,α-Trifluor-m-tolyl)-nicotinsäure
N-(2,3-Xylidino)-nicotinsäure
2-(2-Methyl-3-trifluormethylanilino)-nicotinsäure
N-(3-Chlor-o-tolyl)-nicotinsäure
2-(2,6-Xylidino)-nicotinsäure
2-(2-Hydroxyethoxy)-ethyl-N-(α,α,α-trifluor-n-tolyl)-anthranilat
Ethoxymethyl-N-(2,6-dichloro-m-tolyl)-anthranilat
Butyl-(α,α,α-trifluor-m-tolyl)-anthranilat
Ethyl-(α,α,α-trifluor-m-tolyl)-anthranilat
Isopropyl-(α,α,α-trifluor-m-tolyl)-anthranilat.

4. Depotzubereitung nach den Ansprüchen 1 bis 3, enthaltend als Medium eine oder mehrere Verbindungen aus der Gruppe:
Viscoleo, Isopropylmyristat, Ethyloleat, Ricinusöl, Sesamöl, Arachisöl, Baumwollsamenöl, Mandelöl, Olivenöl, Klauenöl, Neutralöl und Maisöl.

5. Depotzubereitung nach den Ansprüchen 1 bis 4, dadurch gekennzeichnet, daß sie 3 bis 80 Gew.-% Wirkstoff und 2 bis 90 Gew.-% des Mediums, bezogen auf die Zubereitung, enthält.

6. Verwendung der Wirkstoffe der Formel I zur Herstellung von Depotzubereitungen nach den Ansprüchen 1 bis 5 zur Behandlung von entzündlichen und/oder schmerzhaften Erkrankungen.

**Claims**

1. Depot formulations for intramuscular use containing at least one active compound of the formula

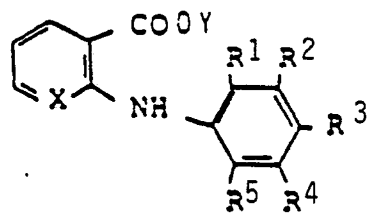

in which
$R^1$-$R^5$ represent hydrogen, alkyl with 1 to 4 C atoms or trihalogenoalkyl,
X represents nitrogen or a CH group and
Y represents hydrogen, metal ions, alkyl with 1 to 4 C atoms, alkoxy, alkoxyalkyl, hydroxy alkyl, hydroxyalkoxyalkyl or trihalogenoalkyl, in which the number of C atoms is in each case between 1 and 6 and the alkyl chain has 1 to 4 C atoms and can be straight-chain or branched,
and, as medium, one or more compounds from the group consisting of mono-, di- and triglycerides with mono-, di- and tricarboxylic acids of $C_2$ to $C_{30}$ chain length, in saturated or unsaturated, and optionally also hydroxylated, form and/or esters of monohydric or polyhydric alcohols of $C_2$ to $C_{30}$ chain length with the said acid components.

2. Depot formulations according to Claim 1, containing at least one active compound of the formula according to Claim 1,
in which
$R^3$ and $R^4$ represent hydrogen,
$R^1$, $R^2$ and $R^5$ represent methyl, trifluoromethyl or chlorine
X represents a CH group and
Y represents hydrogen, metal ions, alkyl with 1 to 4 C atoms or substituted alkyl.

3. Depot formulation according to Claim 1, containing as active compound a compound from the group:
N-(α,α,α-trifluoro-m-tolyl)-anthranilic acid,
N-(2,3-xylyl)-anthranilic acid,
N-(2,6-dichloro-m-tolyl)-anthranilic acid,
N-(3-chloro-o-tolyl)-anthranilic acid,
N-(2,3-dichlorophenyl)-anthranilic acid,
N-(α,α,α,tolyl)-nicotinic acid,
N-(2,3-xylidino)-nicotinic acid,
2-(2-methyl-3-trifluoromethylanilino)-nicotinic acid,
N-(3-chloro-o-tolyl)-nicotinic acid,
2-(2,6-xylidino)-nicotinic acid,
2-(2-hydroxyethoxy)-ethyl-, N-(α,α,α,trifluoro-m-tolyl)-anthranilate,
ethoxymethyl N-(2,6-dichloro-o-tolyl)-anthranilate,
butyl (α,α,α-trifluoro-m-tolyl)-anthranilate,
ethyl (α,α,α-trifluoro-m-tolyl)-anthranilate and
isopropyl (α,α,α-trifluoro-m-tolyl)-anthranilate.

4. Depot formulation according to Claims 1 to 3, containing as medium one or more compounds from the group: viscoleo, isopropoyl myristate, ethyl oleate, castor oil, sesame oil, arachis oil, cottonseed oil, almond oil, olive oil, neat's foot oil, neutral oil and maize oil.

5. Depot formulation according to Claims 1 to 4, characterized in that it contains 3 to 80 % by weight of active compound and 2 to 90 % by weight of the medium, relative to the formulation.

6. Use of the active compounds of the formula I for the preparation of depot formulations according to Claims 1 to 5 for treating inflammatory and/or painful diseases.

**Revendications**

1. Préparations à action prolongée pour l'administration intramusculaire, contenant au moins une substance active de formule

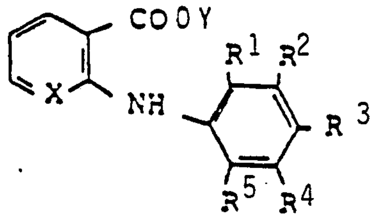

dans laquelle

$R^1$ à $R^5$ représentent l'hydrogène, des groupes alkyle ayant 1 à 4 atomes de carbone, des groupes trihalogénalkyle,

X représente l'azote ou un groupe CH et

Y représente l'hydrogène, des ions métalliques, des groupes alkyle ayant 1 à 4 atomes de carbone, alkoxy, alkoxyalkyle, hydroxyalkyle, hydroxyalkoxyalkyle, trihalogénalkyle, le nombre d'atomes de carbone étant situé entre 1 et 6 dans chaque cas et la chaîne alkylique présentant 1 à 4 atomes de carbone et pouvant être droite ou ramifiée,

et comme milieu, un ou plusieurs composés du groupe des mono-, di- et triglycérides avec des acides mono-, di- et tricarboxyliques de longueur de chaîne allant de $C_2$ à $C_{30}$, sous la forme saturée de même que sous la forme insaturée, et aussi le cas échéant sous la forme hydroxylée et/ou sous la forme d'esters d'alcools monovalents ou polyvalents de longueur de chaîne allant de $C_2$ à $C_{30}$ avec les composants acides mentionnés.

2. Préparations à action prolongée suivant la revendication 1, contenant au moins une substance active de formule suivant la revendication 1, dans laquelle

$R^3$ et $R^4$ représentent l'hydrogène,

$R^1$, $R^2$, $R^5$ représentent un groupe méthyle, trifluorométhyle, du chlore,

X est un groupe CH et

Y représente l'hydrogène, des ions métalliques, un groupe alkyle ayant 1 à 4 atomes de carbone ainsi qu'un groupe alkyle substitué.

3. Préparation à action prolongée suivant la revendication 1, contenant comme substance active un composé du groupe:

acide N-(α,α,α-trifluoro-m-tolyl)-anthranilique
acide N-(2,3-xylyl)-anthranilique
acide N-(2,6-dichloro-m-tolyl)-anthranilique
acide N-(3-chloro-o-tolyl)-anthranilique
acide N-(2,3-dichlorophényl)-anthranilique
acide N-(α,α,α-trifluoro-m-tolyl)-nicotinique
acide N-(2,3-xylidino)-nicotinique
acide 2-(2-méthyl-3-trifluorométhylanilino)-nicotinique
acide N-(3-chloro-o-tolyl)-nicotinique
acide 2-(2,6-xylidino)-nicotinique
N-(α,α,α-trifluoro-m-tolyl)-anthranilate de 2-(2-hydroxyéthoxy)-éthyle
N-(2,6-dichloro-m-tolyl)-anthranilate d'éthoxyméthyle
(α,α,α-trifluoro-m-tolyl)-anthranilate de butyle
(α,α,α-trifluoro-m-tolyl)-anthranilate d'éthyle
(α,α,α-trifluoro-m-tolyl)-anthranilate d'isopropyle

4. Préparation à action retardée suivant les revendications 1 à 3, contenant comme milieu un ou plusieurs composés du groupe:

viscoléo, myristate d'isopropyle, oléate d'éthyle, huile de ricin, huile de sésame, huile d'arachide, huile de graine de cotonnier, huile d'amande, huile d'olive, huile de pied de boeuf, huile neutre et huile de mais.

5. Préparation à action retardée suivant les revendications 1 à 4, caractérisée en ce qu'elle contient 3 à 80 % en poids de substance active et 2 à 90 % en poids de milieu, par rapport à la préparation.

6. Utilisation des substances actives de formule I pour l'obtention de préparations à action retardée selon les revendications 1 à 5 pour le traitement de maladies accompagnées d'inflammation et/ou de douleurs.